# EUROPEAN PATENT SPECIFICATION

(11) **EP 1 432 468 B1**
(45) Date of publication and mention of the grant of the patent: **06.08.2008**
(21) Application number: 02758560.3
(22) Date of filing: 02.09.2002
(51) Int. Cl.: A61M 39/10

(54) **COUPLING FOR MEDICAL FLUID DELIVERY SYSTEMS**
KUPPLUNG FÜR MEDIZINISCHE FLÜSSIGKEITSABGABESYSTEME
RACCORD POUR DES SYSTEMES D'ADMINISTRATION DE FLUIDE MEDICAL

(30) Priority: 04.09.2001 GB 0121403
(43) Date of publication of application: 30.06.2004
(73) Proprietor: Clinical Product Development Limited, Herts SG12 8LD (GB)
(72) Inventor: PETERS, Joseph Lennox, Herts SG12 8LD (GB); WATKINSON, John, Herts SG12 8LD (GB)
(74) Representative: Lerwill, John
(86) International application number: PCT/GB2002/004008
(87) International publication number: WO 2003/020361

(56) References cited:
- EP-A- 0 633 039
- EP-A- 1 050 318
- WO-A-99/37356
- GB-A- 2 131 510
- US-B1- 6 217 564

## Description

This invention relates to fluid couplings for medical equipment and is directed in particular to a coupling suitable for use in performing spinal injections.

It has been the practice for the same form of couplings to be used for delivery for drugs when carrying out vascular and spinal injections, and as a result there is a risk of drugs being incorrectly administered with serious consequences for the patient. Fatal accidents have occurred due to such mistakes, in particular with improper drugs being injected into the spine. There is, therefore a need for a coupling which can be dedicated for spinal use and which is adapted to provide a variety of safeguards to help avoid such accidents occurring. In addition to looking to fulfil this need the invention disclosed herein aims to provide a fluid coupling of compact form in which the coupled parts are effectively secured against accidental separation, and can have indicators providing distinctive visual and tactile features enabling the components to be readily identified and distinguished from others intended for different uses, or at least not meant to be used with the components of the coupling of the invention.

In WO/95 22369 there is described a medical coupling in which a male member has a tapered spigot which fits into a correspondingly shaped socket provided in a hub of the female member. To secure the members against axial separation, a cap on the male member is threadedly engaged with the exterior of the hub. The cap is locked against unintentional release by a ratchet and pawl mechanism, there being ratchet teeth disposed around the female member forwardly of the hub and the cap having a forward extension with a ring on which the pawl is carried. This arrangement of the ratchet and pawl mechanism results in an elongation of the coupling members. EP-A-0633038 describes a generally similar form of coupling, the cap in this case being extended forwardly by a sleeve carrying the pawls which cooperate with a single pair of radial webs. In addition to the coupling components being axially extended by the locking arrangement, a pair of pawls cooperating with just two diametrically opposite webs can not guarantee effective locking of the cap in its final tightening position.

GB-A-2131510 discloses a medical coupling in which the male and female members are equipped with cage-like guards to reduce the risk of contamination by contact, but there is no mechanism to lock the cap against becoming loosened.

In EP-A-0633039 there is described a medical coupling including a female part having a socket recess with an inner section that tapers inwardly away from the open end of the socket for sealing cooperation with a male connector which is provided with radially projecting pins for engagement with bayonet slots in an outer end portion of the hub to secure the male and female members together.

In accordance with the present invention there is provided a female coupling member for a medical fluid delivery system intended for administrating liquids to a patient as defined in claim 1.

Medical couplings of the Luer type having interengagable spigot and socket parts are currently manufactured to an international standard setting down the taper angle and diameters of these parts. These couplings are very widely used, for vascular cannulae. The preferred form of female couplings of the present invention is adapted for spinal drug administration and has a socket opening into which the conventional Luer spigot of currently used dimensions will not fit. If attempt is made to deliver liquid into the socket of the female coupling by means of a male part of the known larger dimensions, e.g. located at the forward end of a syringe, being applied against the end of the hub, the liquid will leak out through the escape vent opening due to the relatively high back pressure encountered when performing spinal injections and the erroneous attempt to inject an inappropriate drug into the spine will fail.

A preferred form of fluid coupling for a medical fluid delivery system intended for administrating liquids to a patient, such as a spinal injection system, includes the female member of the invention and a male member having a tubular spigot insertable into the socket for sealing cooperation with the interior surface of the socket recess, and a collar extending about the spigot for surrounding the hub when the spigot is inserted into the socket, the collar and hub having complementary securing means engageable by relative rotation of the hub and collar for securing the male and female members against axial disengagement; wherein the female member has a sleeve surrounding the hub for extending around the collar when the spigot is inserted into the socket, and the collar has a series of several ratchet teeth disposed around the outer surface thereof for cooperation with a ratchet pawl element provided on the inner surface of the sleeve to lock the collar against rotation in a direction to disengage the securing means, and the pawl element being displaceable to disengage the ratchet teeth by squeezing and resiliently deforming the sleeve.

With the ratchet teeth of the locking mechanism being located on the collar and the pawl element being on a sleeve surrounding the hub of the female member, an axially compact construction is achieved.

In a preferred fluid coupling, the sleeve has one or more openings which, in addition to possibly providing an escape route for any improperly delivered liquids, allows exposure of the parts of the coupling located within the sleeve to a sterilizing treatment, such as by ultra violet light or other electromagnetic radiations having a property of destroying bacteria, or application of a fluid delivered by an aerosol spray. The sleeve has two pawl elements located diametrically opposite each other and the outer surface of the sleeve has raised portions at the areas where the sleeve should be squeezed to disengage the pawl element(s) from the ratchet teeth. The sleeve is circular in profile and is radially spaced from the ratchet teeth to enable disengagement of the pawl element(s) from the teeth.

The female member in the preferred embodiment has a narrow end portion remote from the hub, and several, e.g. 5 to 7, radially projecting elements, referred to hereinafter as wings, are spaced apart around the narrow end portion and protrude outwardly from the surface of the female member at a gripping region defined adjacent the wings and axially inwardly thereof. The wings give the female member a distinctive visual appearance enabling it to be differentiated from other forms of female couplings. Furthermore, these wings give the female member a distinctive feel and hence a tactile indication of the type of coupling being handled. The wings provide an abutment for the fingers to press against, e.g. when inserting a catheter needle attached to the member through body tissue, and the user will recognise the type of coupling member from the distinctive feel of the wings. For the same reasons of providing distinctive visual and tactile indications, the male member is also provided with radially extending wings, equal in number and circumferential spacing to the wings on the female member, extending along the male member behind the spigot and collar. However, the wings on the male member also assist gripping during connection and disconnection.

The male coupling member may comprise a syringe with the wings being located axially forwardly of the front end of the syringe barrel. More particularly the wings can be connected to the front end wall of the syringe barrel and have outer edges which are contoured to follow the shape of a dome, the wings terminating at a flange from which the spigot and collar extend forwardly. Opposed gripping lugs may project outwardly from the barrel adjacent the read end and further wings with forward ends attached to the lugs can project radially from the barrel behind the lugs. Additional visual and tactile indications of the kind of syringe are provided by the piston having a push plate with a lobed periphery. The wings on the syringe, e.g. at the forward end of the barrel, can give the syringe a shape preventing its use with driving or pumping devices currently in use with known syringes for gradual delivery of drugs in vascular injection systems.

From the foregoing description it will be appreciated that the present invention provides coupling members suited for a particular use to which they are dedicated and enabling easy identification by touch and by visual inspection as well as incorporating further safeguards to ensure proper and reliable use. A clear understanding of the invention will be gained from the following detailed description of a preferred coupling system according to the invention, reference being made to the accompanying drawings in which:
Figure 1 is an isometric view of the female member;
Figure 2 is an isometric view of a male member for forming an in-line coupling with the female member;
Figure 3 is an isometric view of the assembled in-line coupling;
Figure 4 is a side elevation of the in-line coupling;
Figure 5 is a plan of the in-line coupling;
Figure 6 is an end elevation of the in-line coupling;
Figure 7 is an axial section through the in-line coupling;
Figures 8A, 8B and 8C are side, plan and perspective views respectively, showing the female member in combination with a stylet holder;
Figure 9 is an enlarged axial cross-section through the female member and stylet holder combination of Figure 8;
Figure 10 is an exploded isometric view of the female member and a syringe adapted for use therewith;
Figures 11A, 11B and 11C are plan, side and perspective view respectively, showing the female member and syringe coupled together;
Figure 12 is an axial cross-section taken along the line XII-XII in Figure 11; and
Figure 13 is a transverse cross-section taken along the line XIII-XIII in Figure 11.

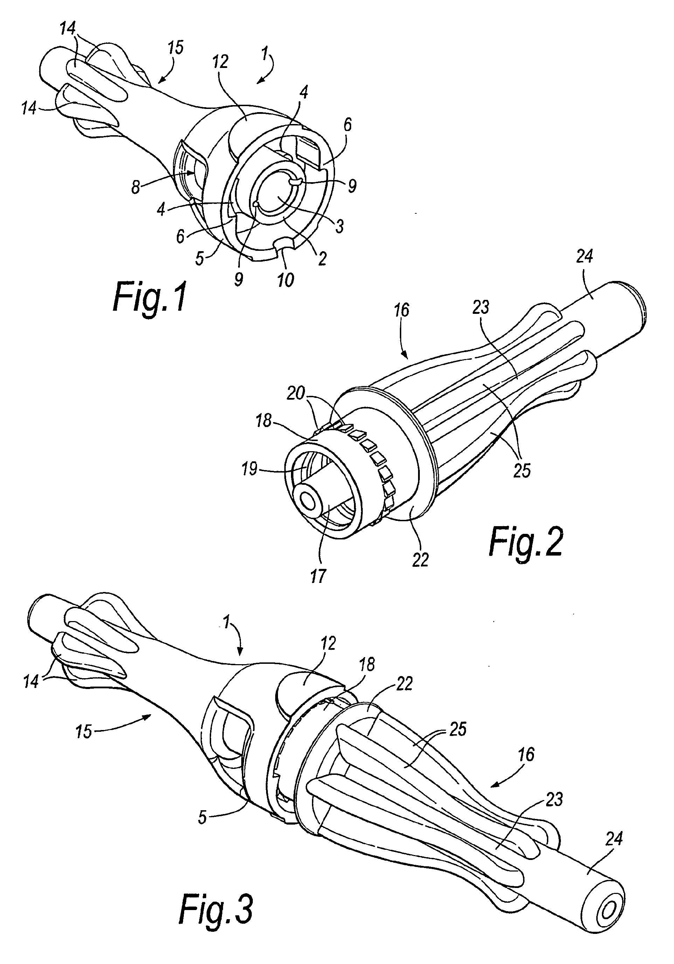

Illustrated in the drawings and described below are fluid coupling members and couplings specifically intended for spinal infusion injection therapy access and adapted to be differentiated and easily distinguishable from known vascular administration systems. The female coupling member 1, shown in Figure 1, is made as a one piece plastics moulding and includes a hub 2 with an inwardly tapering socket 3. On the exterior of the hub are helical screw thread segments 4. A sleeve 5 is integral with the hub 2 at its forward end and extends rearwardly to an open end level with the rear end of the hub 2. Provided on the interior of the sleeve 5 adjacent the rear end are a pair of diametrically opposed inwardly projecting pawls 6 for cooperation with the ratchet teeth of a ratchet and pawl locking mechanism, as will become clear. The sleeve 5 has window apertures or openings 8, two as shown, intermediate its ends and providing access to the space within the sleeve 5 around the hub 2. At the open end of the socket 3, the hub 2 is provided with a pair of small axial grooves or notches 9, located diametrically opposite each other. The notches 9 form escape vents through which liquid can escape if supplied into the socket through an incorrectly fitted or inappropriate male member. The rim of the sleeve 5 has notch 10 which is intended to serve to rotationally align a stylet holder, which is desirable if the female member has a hollow needle attached and through which a stylet is intended to be inserted when introducing the needle through body tissue of a patient. Semi-circular raised areas 12 are provided on the exterior of the sleeve adjacent the open end and at positions at 90° to the locations of the pawls 6. The raised areas 12 provide visual and tactile indicators of the positions at which the sleeve 5 should be squeezed, e.g. between the thumb and forefinger, for disengaging the pawls 6 from the ratchet teeth of a male member engaged with the female member 1.

Forwardly from the hub 2 and sleeve 5, the female member tapers gradually and smoothly to a narrow end portion at which several radially projecting fins or wings 14 are uniformly spaced apart around the member. The wings 14 combined with the surface of the female member directly behind the wings to define a waisted gripping region 15 at which the member 1 is intended to be held when introducing a needle (not shown) attached to and projecting forwardly from the female coupling member, through body tissue into the spinal cavity. The wings 14 thereby provide a highly distinctive visual and tactile indication that it is a spinal female coupling member, as well as providing an abutment for the fingers to press against during needle insertion. A suitable number of wings 14 is 5 to 7 with 6 being presently preferred as shown in the drawings.

The female coupling member, can be connected with the male coupling member 16 shown in Figure 2 to form an in-line coupling, e.g. for connecting a fluid supply tube to the female member. The male member 16 is also made as a one piece plastic moulding. It comprises a tapered tubular spigot 17 shaped and dimensioned to fit into the socket 3 of the female member and to form a sealed connection therewith. The male member has a collar 18 coaxial with and spaced outwardly from the spigot 17 and a screw thread 19 is provided on the interior of the collar 18 for cooperation with the thread segments 4 on the hub 2 of the female member. When the male and female members are brought together, the spigot 17 is introduced into socket 3 and the two members 1, 16 are relatively rotated so that the thread 19 and thread segments 4 engage and urge the spigot 17 into tight sealing engagement with the tapered socket surface, and also secure the coupled members against being pulled axially apart. Positioned in a ring around the exterior of the collar are a large number of ratchet teeth 20 with which the pawls 6 on the sleeve 5 cooperate when the male and female members 1, 16 are connected together. The pawls 6 and ratchet teeth 20 are shaped so that the pawls ride over and click past the teeth 20 as the collar 18 is tightened onto the hub 2 by the threaded connection, but the pawls 6 engage the teeth 20 to lock the collar 18 against rotation in the loosening direction. A sufficient radial clearance is provided between the inner surface of the sleeve 5 and the extremities of the ratchet teeth 20 so that when the sleeve is squeezed at the areas 12 the sleeve can be deformed enough to disengage the pawls 6 from the ratchet teeth 20 and thereby allow rotation of the collar to disconnect the male and female members. The spigot 17 and collar 18 project forwardly from a flange 22 and behind this flange the male member has a tubular core 23 with a connection piece 24 at the end for attachment of a supply tube (not shown), and a plurality of radial wings 25, equal in number and in circumferential spacing to the wings 14 on the female member 1. The wings 25 assist gripping to facilitate rotation of the male member 16 in connecting this member to and disconnecting it from the female member 1, and they also give the male member a distinctive visual appearance and feel to facilitate recognition and differentiation from the male members of other forms of coupling.

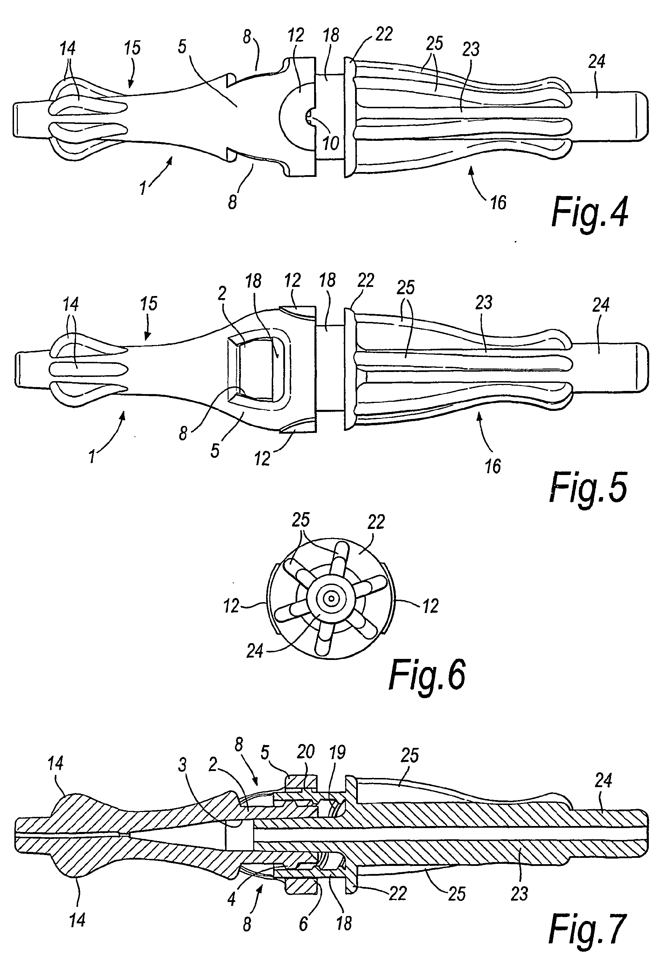

The assembled in-line coupling is illustrated in Figures 3-7. The window openings 8 allow inner parts of the coupling to be treated to deter bacterial colonisation. In particular ultra violet light can be shone through the openings, e.g. from a u.v. pen torch, and be transmitted within the coupling by reflection. Alternatively other electromagnetic radiation having the property of killing bacteria can be used, or a liquid bactericide can be applied such as in the form of an aerosol spray.

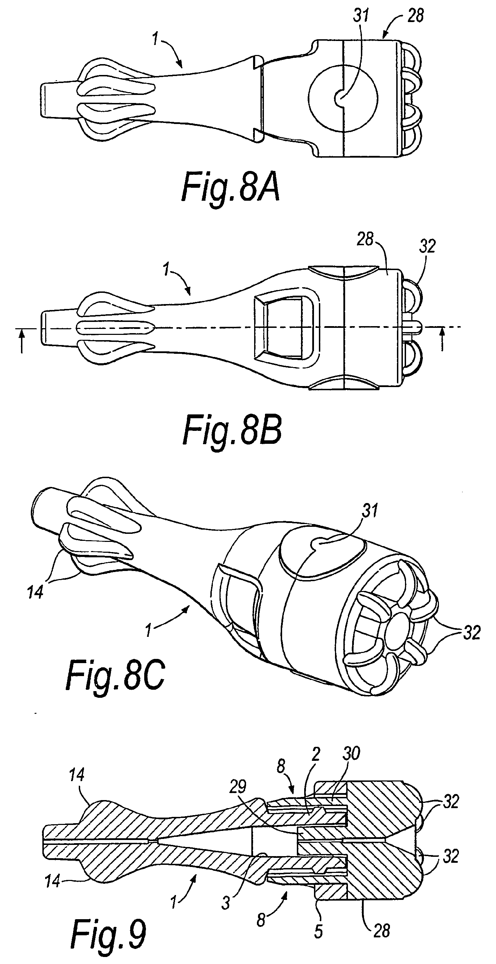

Figures 8 and 9 show the female coupling member 1 in combination with a stylet holder 28. The stylet holder 28 has a generally similar form to the front end of the male member 16 with a spigot 29 and a collar 30. The spigot 29 is insertable into the socket 3 for alignment purposes, but it does not make sealing contact with the socket surface. The collar 30 slides axially between the hub 2 and the sleeve 5 and it is not provided with any formations intended to interengage with either thread segments 4 or the pawls 6. However, axially extending grooves may be formed in the inside of the collar to receive the thread segments 4 and thereby provide some rotational alignment between the stylet 28 and the female member. The stylet holder has a forwardly projecting nib 31 which engages in the notch 10 in the sleeve 5 to ensure correct rotational alignment between the stylet holder 28 and the female member 1. The stylet is not shown in the drawings, but it will be understood that it will be fixed to extend axially forwardly from stylet holder 28 through a hollow needle attached to the female member. The rear face of the stylet holder, where pressure is usually applied by means of a finger or thumb when introducing the stylet tip and needle into the body tissue, has a set of uniformly spaced radial lugs 32 which project rearwardly. The number of lugs 32 is preferably the same as the number of wings 14 on the female member, i.e. 6, and these lugs ensure a distinctive appearance and feel to facilitate precise recognition of the type of equipment.

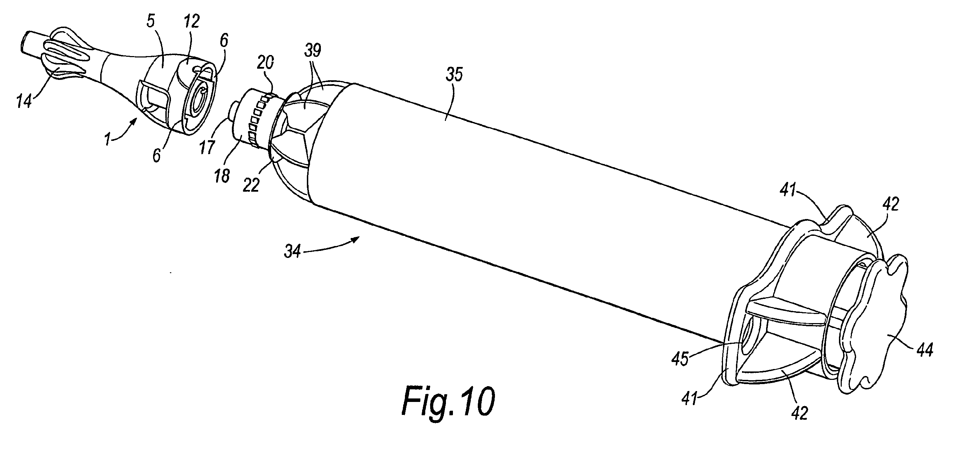

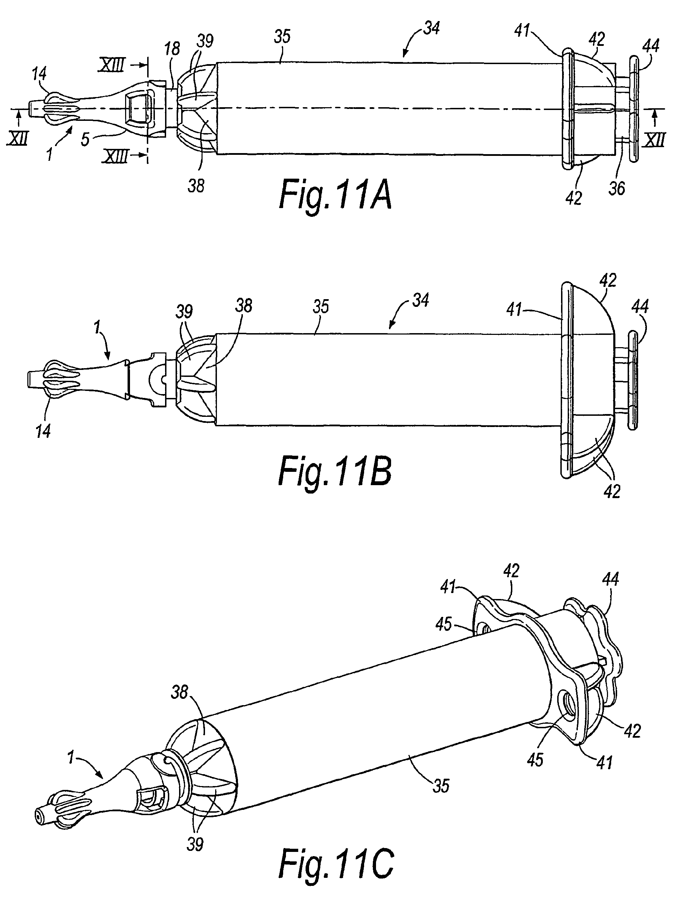

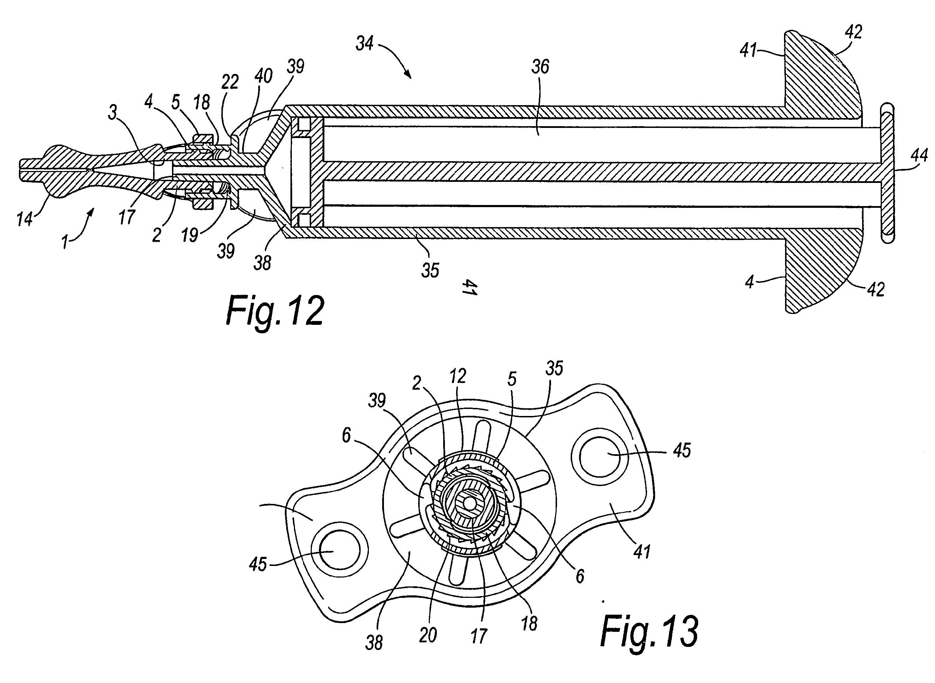

The female coupling member 1 is shown in combination with a syringe 34 in Figures 10 to 13. The syringe comprises a barrel 35 and a piston 36. A male coupling member 37 is integral with the front end wall 38 of the barrel and includes an spigot 17 and collar 18 extending forwardly from a flange 22, these parts having exactly the same form as the corresponding parts of the male coupling member 16 shown in Figure 2, for connecting the syringe to the female coupling member 1. A plurality of radial wings 39 are positioned between the front end wall 38 of the barrel and the flange, the wings 39 being uniformly distributed around the discharge tube 40 of the syringe. The number of wings 39 is approximately and preferably exactly the same as the number of wings 14 on the female member for consistency of appearance and feel to facilitate recognition of the fact that the parts are adapted for use with one another, there being six wings 39 on the syringe shown in the drawings. The outer edges of the wings 39 follow the shape of a dome between the front end wall 38 and the flange 22. Integral with the syringe barrel adjacent its rear end are a pair of diametrically opposed lugs 41 to enable the barrel 35 to be gripped when driving the piston 36 forwardly for injecting the syringe contents. Further radial wings 42 which reinforce the lugs 41 are positioned behind the lugs 41, these wings 42 also having outer edges which follow the shape of a dome between the lugs 41 and the end of the barrel 35 so as to assist correct identification of the form of syringe and thereby the nature of its contents. Additional visual and tactile indications of the syringe type are provided by the piston 36 having a push plate 44 with a distinctive peripheral shape, in particular with six radial lobes, and the gripping lugs 41 having holes 45 in the areas where they are normally contacted by the fingers of a user holding the syringe ready for discharging its contents. The way in which the syringe 34 is connected to and disconnected from the female coupling member 1 will be understood from the description of the in-line coupling given above.

From the foregoing description it will be understood that the coupling members of the invention are uniquely adapted for use together and embody many features to ensure safe and reliable use as well as to provide clear visual and tactile indicators to enable users to identify the equipment being handled with certainty and without risk of error. These features and attributes in combination result in couplings which are ideally suited for adoption in equipment intended for performing spinal injection procedures as distinct from other types of injections.

## Claims

1. A female coupling member for a medical fluid delivery system intended for administrating liquids to a patient, comprising a hub (2) with a socket recess (3) having an open end and an interior surface tapering inwardly from the open end for sealing cooperation with a complementary tubular male connector (16), a securing element (4) on the exterior of the hub (2) for cooperation with a complementary securing means on a collar (18) attached to the complementary male connector for securing the female member against axial disengagement from a complementary male connector engaged therewith, and **characterised in that** a venting means is provided on the hub (2) to form a liquid escape vent (9) open to the tapering surface adjacent the open end for conducting to the exterior of the hub liquid introduced into the socket recess (3) by a supply member presented to the open end, but without the supply member having sealing engagement with the socket surface at a location inwardly of the escape vent (9), the venting means comprising one or more grooves or notches in the interior surface extending inwardly from the open end, and/or one or more radial through holes adjacent the open end, and/or one or more through slots at the open end.

2. A female coupling member according to claim 1, wherein a plurality of escape vents (9) are distributed around the circumference of the socket recess.

3. A fluid coupling for a medical fluid delivery system intended for administrating liquids to a patient, such as a spinal injection system, comprising:
a female member (1) as defined in claim 1 or 2; and
a male member (16) having a tubular spigot (17) insertable into the socket (3) for sealing cooperation with the interior surface of the socket recess and a collar (18)
extending about the spigot (17) for surrounding the hub (2) when the spigot is inserted into the socket, the collar (18) and hub (2) having complementary securing means (4, 19) engageable by relative rotation of the hub and collar for securing the male and female members against axial disengagement;
wherein
the female member (1) has a sleeve (5) surrounding the hub (2) for extending around the collar (18) when the spigot is inserted into the socket, and the collar (18) has a series of several ratchet teeth (20) disposed around the outer surface thereof for cooperation with a ratchet pawl element (6) provided on the inner surface of the sleeve (5) to lock the collar against rotation in a direction to disengage the securing means, and the pawl element (6) being displaceable to disengage the ratchet teeth by squeezing and resiliently deforming the sleeve (5).

4. A coupling according to claim 3, wherein the outer surface of the sleeve (5) has raised portions (12) at the areas where the sleeve should be squeezed to disengage the pawl element (6).

5. A coupling according to claim 3 or 4, wherein the sleeve (5) has two pawl elements (6) at diametrically opposite portions.

6. A coupling according to claim 3, 4 or 5, wherein the sleeve (5) has at least one opening (8) through which the parts of the coupling located within the sleeve may be subjected to a sterilization treatment, such as by application of ultra violet light or other electromagnetic radiation capable of destroying bacteria or by spraying with an aerosol bactericide.

7. A female coupling member according to claim 1 or 2, or a coupling according to any one of claims 3 to 6, wherein the female member (1) has a narrow end portion remote from the socket, and several radially projecting wings (14) are spaced apart around the narrow end portion and protrude outwardly beyond the surface of the female member at a gripping region (15) defined immediately adjacent and axially inwardly of the wings.

8. A female coupling member or a coupling according to claim 7, wherein the number of wings (14) is from 5 to 7.

9. A coupling according to claim 7 or 8, wherein the male member (16) has radial wings (25) extending axially therealong behind the collar and spigot.

10. A coupling according to claim 9, wherein the wings (25) on the male member (16) are equal in number and spacing to the wings (14) on the female member (1).

11. A coupling according to claim 9 or 10, wherein the male member comprises a syringe (34), including a barrel (35) and a piston (36), and the wings (39) are located axially forwardly of a front end wall of the barrel (35).

12. A coupling according to claim 11, wherein the wings (39) extend axially from the front end wall of the barrel to a flange (22) from which the spigot and collar extend forwardly.

13. A coupling according to claim 11 or 12, wherein opposed gripping lugs (41) extend outwardly from the barrel (35) adjacent the rear end of the barrel, and further wings (42) with forward ends attached to the lugs (41) project radially from the barrel behind the lugs.

14. A coupling according to claim 11, 12 or 13, wherein the piston (36) has a push plate (44) with a non-circular peripheral profile, e.g. with radial lobes, at the outer end of the piston.

15. A female coupling member according to claim 1 or 2, wherein a sleeve (5) is connected to forward end of the hub (2) and extends rearwardly therefrom, the sleeve including an opening (8) through which the interior of the sleeve and the hub can be subjected to treatment for destroying bacteria, and a locking pawl on the interior of the sleeve for cooperation with complementary locking teeth on the male connector.

16. A fluid coupling for a medical fluid delivery system for administering liquids to a patient, comprising a female coupling member according to claim 1 or 2, and a male coupling member (16) including a tubular spigot (17) for insertion into the socket (3) of the female coupling member, a collar (18) coaxially surrounding the spigot, a screw thread (19) on the interior of the collar (18), and a large number of ratchet teeth (20) disposed around the exterior of the collar and shaped for cooperation with a locking pawl (6) to permit rotation of the collar in a direction to tighten the collar onto a complementary threaded part (4) of the hub of the female member and to lock the collar against rotation in the opposite direction.

17. A female coupling member according to claim 1, 2, or 15 in combination with a stylet holder (28) having a rear face for applying pressure to the stylet holder during needle insertion, the rear face having radial lugs (32) protruding axially therefrom.

## Patentansprüche

1. Aufnahmekupplungselement für ein medizinisches Flüssigkeitsabgabeelement, das zum Verabreichen von Flüssigkeiten an einen Patienten konzipiert ist und eine Nabe (2) mit einer Buchsenaussparung (3) mit einem offenen Ende und einer Innenfläche, die sich von dem offenen Ende nach innen verjüngt, zur abdichtenden Zusammenwirkung mit einem komplementären rohrförmigen Stecker (16), ein Sicherungselement (4) auf der Außenseite der Nabe (2) zur Zusammenwirkung mit einem komplementären Sicherungsmittel auf einem Bund (18), der an dem komplementären Stecker angebracht ist, zum Sichern des Aufnahmeelements gegen eine axiale Auskupplung von einem darin eingreifenden komplementären Stecker umfasst und **dadurch gekennzeichnet, dass** ein Abzugsmittel auf der Nabe (2) vorgesehen ist, um eine Flüssigkeitsableitungsöffnung (9) zu bilden, die zu der sich verjüngenden Fläche hin angrenzend an das offene Ende offen ist, zum Leiten von Flüssigkeit, die von einem Zufuhrelement, das zu dem offenen Ende dargeboten ist, in die Buchsenaussparung (3) eingeführt wurde, zu der Außenseite der Nabe, wobei das Zufuhrelement jedoch keinen abdichtenden Eingriff mit der Buchsenoberfläche an einer Stelle, die zu der Ableitungsöffnung (9) innen liegt, aufweist, wobei das Abzugsmittel eine oder mehrere Nuten oder Kerben in der Innenfläche, die sich von dem offenen Ende nach innen erstrecken, und/oder ein oder mehrere radiale Durchgangslöcher angrenzend an das offene Ende und/oder ein oder mehrere Durchgangsschlitze an dem offenen Ende umfasst.

2. Aufnahmekupplungselement nach Anspruch 1, in dem mehrere Ableitungsöffnungen (9) um den Umfang der Buchsenaussparung herum verteilt sind.

3. Flüssigkeitskupplung für ein medizinisches Flüssigkeitsabgabesystem, das zum Verabreichen von Flüssigkeiten an einen Patienten konzipiert ist, wie ein Spinalinjektionssystem, die Folgendes umfasst:
ein Aufnahmeelement (1), wie in Anspruch 1 oder 2 definiert; und
ein Steckelement (16) mit einem rohrförmigen Zapfen (17), der in die Buchse (3) eingesetzt werden kann, zur abdichtenden Zusammenwirkung mit der Innenfläche der Buchsenaussparung und einem Bund (18), der sich um den Zapfen (17) herum erstreckt, zum Umgeben der Nabe (2), wenn der Zapfen in die Buchse eingesetzt wird, wobei der Bund (18) und die Nabe (2) komplementäre Sicherungsmittel (4, 19) aufweisen, die durch relative Drehung der Nabe und des Bunds eingekuppelt werden können, zum Sichern des Aufnahmeelements und des Steckelements gegen eine axiale Auskupplung; in der
das Aufnahmeelement (1) eine Muffe (5) aufweist, die die Nabe (2) umgibt, zum Erstrecken um den Bund (18) herum, wenn der Zapfen in die Buchse eingesetzt wird, und der Bund (18) eine Reihe mehrerer Sperrzähne (20) aufweist, die um die Außenfläche dieses herum angeordnet sind, zur Zusammenwirkung mit einem Sperrklinkenelement (6), das auf der Innenfläche der Muffe (5) vorgesehen ist, um den Bund gegen Drehung in einer Richtung zu arretieren, um das Sicherungsmittel auszukuppeln, und das Klinkenelement (6) versetzt werden kann, um die Sperrzähne auszukuppeln, indem die Muffe (5) zusammengedrückt und elastisch verformt wird.

4. Kupplung nach Anspruch 3, in der die Außenfläche der Muffe (5) erhabene Abschnitte (12) an den Bereichen, an denen die Muffe zusammengedrückt werden sollte, um das Klinkenelement (6) auszukuppeln, aufweist.

5. Kupplung nach Anspruch 3 oder 4, in der die Muffe (5) zwei Klinkenelemente (6) an genau entgegengesetzten Abschnitten aufweist.

6. Kupplung nach Anspruch 3, 4 oder 5, in der die Muffe (5) mindestens eine Öffnung (8) aufweist, durch die die Teile der Kupplung, die in der Muffe angeordnet sind, einer Sterilisationsbehandlung unterzogen werden können, wie durch Anwendung von ultraviolettem Licht oder anderer elektromagnetischer Strahlung, die Bakterien zerstören kann, oder durch Besprühen mit einem vernebelten Bakterizid.

7. Aufnahmekupplungselement nach Anspruch 1 oder 2 oder Kupplung nach einem der Ansprüche 3 bis 6, in dem bzw. der das Aufnahmeelement (1) einen schmalen Endabschnitt aufweist, der sich von der Buchse entfernt befindet, und mehrere sich radial erstreckende Flügel (14) um den schmalen Endabschnitt herum beabstandet sind und sich nach außen über die Oberfläche des Aufnahmeelements hinaus an einem Haltebereich (15), der direkt angrenzend an die Flügel und axial nach innen zu den Flügeln definiert ist, erstrecken.

8. Aufnahmekupplungselement oder Kupplung nach Anspruch 7, in dem bzw. der die Anzahl der Flügel (14) von 5 bis 7 ausmacht.

9. Kupplung nach Anspruch 7 oder 8, in der das Steckelement (16) radiale Flügel (25) aufweist, die sich axial daran entlang hinter dem Bund und dem Zapfen erstrecken.

10. Kupplung nach Anspruch 9, wobei die Flügel (25) auf dem Steckelement (16) hinsichtlich der Anzahl und Beabstandung den Flügeln (14) auf dem Aufnahmeelement (1) entsprechen.

11. Kupplung nach Anspruch 9 oder 10, in der das Steckelement eine Spritze (34) umfasst, die einen Zylinder (35) und einen Kolben (36) beinhaltet, und die Flügel (39) axial nach vorne zu einer Vorderendwand des Zylinders (35) angeordnet sind.

12. Kupplung nach Anspruch 11, in der die Flügel (39) sich axial von der Vorderendwand des Zylinders zu einem Flansch (22) erstrecken, von dem sich der Zapfen und der Bund nach vorne erstrecken.

13. Kupplung nach Anspruch 11 oder 12, in der sich entgegengesetzte Halteansätze (41) nach außen von dem Zylinder (35) angrenzend an das hintere Ende des Zylinders erstrecken und weitere Flügel (42) mit vorderen Enden, die an den Ansätzen (41) angebracht sind, radial von dem Zylinder hinter den Ansätzen hervorstehen.

14. Kupplung nach Anspruch 11, 12 oder 13, in der der Kolben (36) eine Schiebeplatte (44) mit einem nicht kreisförmigen Umfangsprofil, z. B. mit radialen Nasen, an dem äußeren Ende des Kolbens aufweist.

15. Aufnahmekupplungselement nach Anspruch 1 oder 2, in dem eine Muffe (5) mit dem vorderen Ende der Nabe (2) verbunden ist und sich von diesem nach hinten erstreckt, wobei die Muffe eine Öffnung (8), durch die die Innenseite der Muffe und der Nabe einer Behandlung zum Zerstören von Bakterien unterzogen werden kann, und eine Arretierklinke auf der Innenseite der Muffe zur Zusammenwirkung mit komplementären Arretierzähnen auf dem Stecker beinhaltet.

16. Flüssigkeitskupplung für ein medizinisches Flüssigkeitsabgabesystem zum Verabreichen von Flüssigkeiten an einen Patienten, die ein Aufnahmekupplungselement nach Anspruch 1 oder 2 und ein Steckkupplungselement (16) umfasst, das einen rohrförmigen Zapfen (17) zum Einsetzen in die Buchse (3) des Aufnahmekupplungselements, einen Bund (18), der den Zapfen koaxial umgibt, ein Schraubgewinde (19) auf der Innenseite des Bunds (18) und eine große Anzahl Sperrzähne (20), die um die Außenseite des Bunds herum angeordnet sind und zur Zusammenwirkung mit einer Arretierklinke (6) geformt sind, um eine Drehung des Bunds in einer Richtung zu ermöglichen, um den Bund auf einem komplementären, mit Gewinde versehenen Teil (4) der Nabe des Aufnahmeelements festzuziehen und den Bund gegen Drehung in der entgegengesetzten Richtung zu arretieren, beinhaltet.

17. Aufnahmekupplungselement nach Anspruch 1, 2 oder 15 in Kombination mit einer Stiletthalterung (28) mit einer hinteren Fläche zum Anwenden von Druck auf die Stiletthalterung während der Nadeleinführung, wobei die hintere Fläche radiale Ansätze (32) aufweist, die von dieser axial hervorstehen.

## Revendications

1. Elément d'accouplement femelle pour un système de fourniture de fluide médical destiné à administrer des liquides à un patient, comprenant une plate-forme (2) avec un retrait de douille (3) ayant une extrémité ouverte et une surface intérieure s'amincissant vers l'intérieur depuis l'extrémité ouverte pour une coopération d'étanchéité avec un connecteur mâle tubulaire complémentaire (16), un élément de fixation (4) sur l'extérieur de la plate-forme (2) pour une coopération avec un moyen de fixation complémentaire sur un collier (18) relié au connecteur mâle complémentaire pour fixer l'élément femelle contre un désengagement axial d'un connecteur mâle complémentaire engagé avec celui-ci, et **caractérisé en ce qu'**un moyen de purge est prévu sur la plate-forme (2) pour former un évent d'échappement de liquide (9) ouvert vers la surface s'amincissant de manière adjacente à l'extrémité ouverte pour conduire vers l'extérieur de la plate-forme du liquide introduit dans le retrait de douille (3) par un élément d'alimentation présenté à l'extrémité ouverte, mais sans que l'élément d'alimentation n'ait d'engagement d'étanchéité avec la surface de douille en un emplacement vers l'intérieur de l'évent d'échappement (9), le moyen de purge comprenant une ou plusieurs rainures ou encoches dans la surface intérieure s'étendant vers l'intérieur depuis l'extrémité ouverte, et/ou un ou plusieurs orifices traversants radiaux adjacents à l'extrémité ouverte, et/ou une ou plusieurs fentes traversantes à l'extrémité ouverte.

2. Elément d'accouplement femelle selon la revendication 1, dans lequel une pluralité d'évents d'échappement (9) sont distribués autour de la périphérie du retrait de douille.

3. Accouplement fluidique pour un système de fourniture de fluide médical destiné à administrer des liquides à un patient, tel qu'un système d'injection rachidien, comprenant :
un élément femelle (1) tel que défini à la revendication 1 ou 2 ; et
un élément mâle (16) ayant un tourillon tubulaire (17) insérable dans la douille (3) pour une coopération d'étanchéité avec la surface intérieure du retrait de douille et un collier (18) s'étendant autour du tourillon (17) pour entourer la plate-forme (2) lorsque le tourillon est inséré dans la douille, le collier (18) et la plate-forme (2) ayant des moyens de fixation complémentaires (4, 19) pouvant s'engager par rotation relative de la plate-forme et du collier pour fixer les éléments mâle et femelle contre un désengagement axial ;
dans lequel
l'élément femelle (1) possède un manchon (5) entourant la plate-forme (2) pour s'étendre autour du collier (18) lorsque le tourillon est inséré dans la douille, et le collier (18) possède une série de plusieurs dents triangulaires (20) disposées autour de la surface externe de celui-ci pour une coopération avec un élément de cliquet (6) prévu sur la surface interne du manchon (5) pour verrouiller le collier contre une rotation dans une direction pour désengager le moyen de fixation, et l'élément de cliquet (6) étant déplaçable pour désengager les dents triangulaires en pressant et déformant le manchon (5) de manière élastique.

4. Accouplement selon la revendication 3, dans lequel la surface externe du manchon (5) possède des portions surélevées (12) au niveau des zones où le manchon doit être pressé pour désengager l'élément de cliquet (6).

5. Accouplement selon la revendication 3 ou 4, dans lequel le manchon (5) possède deux éléments de cliquet (6) au niveau de portions diamétralement opposées.

6. Accouplement selon la revendication 3, 4 ou 5, dans lequel le manchon (5) possède au moins une ouverture (8) à travers laquelle les parties de l'accouplement situées au sein du manchon peuvent être soumises à un traitement de stérilisation, tel que par l'application de lumière UV ou d'un autre rayonnement électromagnétique capable de détruire des bactéries ou en pulvérisant un bactéricide aérosol.

7. Elément d'accouplement femelle selon la revendication 1 ou 2, ou accouplement selon l'une quelconque des revendications 3 à 6, dans lequel l'élément femelle (1) possède une portion d'extrémité étroite à l'écart de la douille, et plusieurs ailes faisant saillie radialement (14) sont espacées autour de la portion d'extrémité étroite et font saillie vers l'extérieur au-delà de la surface de l'élément femelle au niveau d'une région de saisie (15) définie de manière immédiatement adjacente et vers l'intérieur des ailes de manière axiale.

8. Elément d'accouplement femelle ou accouplement selon la revendication 7, dans lequel le nombre d'ailes (14) est de 5 à 7.

9. Accouplement selon la revendication 7 ou 8, dans lequel l'élément mâle (16) possède des ailes radiales (25) s'étendant axialement le long de lui derrière le collier et le tourillon.

10. Accouplement selon la revendication 9, dans lequel les ailes (25) sur l'élément mâle (16) sont égales en nombre et en espacement aux ailes (14) sur l'élément femelle (1).

11. Accouplement selon la revendication 9 ou 10, dans lequel l'élément mâle comprend une seringue (34), comprenant un cylindre (35) et un piston (36), et les ailes (39) sont situées vers l'avant de manière axiale d'une paroi d'extrémité avant du cylindre (35).

12. Accouplement selon la revendication 11, dans lequel les ailes (39) s'étendent axialement depuis la paroi d'extrémité avant du cylindre vers une bride (22) de laquelle le tourillon et le collier s'étendent vers l'avant.

13. Accouplement selon la revendication 11 ou 12, dans lequel des oeillets de saisie opposés (41) s'étendent vers l'extérieur depuis le cylindre (35) de manière adjacente à l'extrémité arrière du cylindre, et des ailes supplémentaires (42) avec des extrémités avant reliées aux oeillets (41) font saillie radialement depuis le cylindre derrière les oeillets.

14. Accouplement selon la revendication 11, 12 ou 13,
dans lequel le piston (36) possède une plaque de poussée (44) avec un profil périphérique non circulaire, par exemple, avec des lobes radiaux, à l'extrémité externe du piston.

15. Elément d'accouplement femelle selon la revendication 1 ou 2, dans lequel un manchon (5) est connecté à l'extrémité avant de la plate-forme (2) et s'étend vers l'arrière depuis celle-ci, le manchon comprenant une ouverture (8) à travers laquelle l'intérieur du manchon et de la plate-forme peut être soumis à un traitement pour détruire des bactéries, et un cliquet de verrouillage sur l'intérieur du manchon pour une coopération avec des dents de verrouillage complémentaires sur le connecteur mâle.

16. Accouplement fluidique pour un système de fourniture de fluide médical pour administrer des liquides à un patient, comprenant un élément d'accouplement femelle selon la revendication 1 ou 2, et un élément d'accouplement mâle (16) comprenant un tourillon tubulaire (17) pour l'insertion dans la douille (3) de l'élément d'accouplement femelle, un collier (18) entourant le tourillon de manière coaxiale, un filetage cylindrique (19) sur l'intérieur du collier (18), et un grand nombre de dents triangulaires (20) disposées autour de l'extérieur du collier et formées pour une coopération avec un cliquet de verrouillage (6) pour permettre la rotation du collier dans une direction pour serrer le collier sur une partie filetée complémentaire (4) de la plate-forme de l'élément femelle et pour verrouiller le collier contre une rotation dans la direction opposée.

17. Elément d'accouplement femelle selon la revendication 1, 2 ou 15, en combinaison avec un porte-stylet (28) ayant une face arrière pour appliquer de la pression au porte-stylet au cours de l'insertion d'aiguille, la face arrière ayant des oeillets radiaux (32) faisant saillie de manière axiale depuis celle-ci.
